# EUROPEAN PATENT SPECIFICATION

(11) **EP 1 768 551 B1**
(45) Date of publication and mention of the grant of the patent: **02.06.2010**
(21) Application number: 05754096.5
(22) Date of filing: 21.06.2005
(51) Int. Cl.: A61B 5/026, G01N 33/49, H04B 10/12

(54) **MEANS AND METHOD FOR DETECTION OF BLOOD LEAKAGE FROM WOUNDS**
MITTEL UND VERFAHREN ZUM NACHWEIS VON BLUTLECKS AUS WUNDEN
MOYEN ET PROCEDE DE DETECTION DU SAIGNEMENT DES PLAIES

(30) Priority: 24.06.2004 SE 0401632
(43) Date of publication of application: 04.04.2007
(73) Proprietor: Redsense Medical Malta Ltd., Msida MSD 1703 (MT)
(72) Inventor: ENGVALL, Daniel, S-302 41 Halmstad (SE); JONSSON, Lennart, S-302 48 Halmstad (SE); KARLSSON, Per-Olof, S-302 52 Halmstad (SE); RUTGERSSON, Lars, S-310 41 Gullbrandstorp (SE); SVENSSON, Fredrik, S-302 49 Halmstad (SE)
(74) Representative: Nunnenkamp, Jörg
(86) International application number: PCT/SE2005/000960
(87) International publication number: WO 2006/001759

(56) References cited:
- WO-A2-01/68163
- WO-A2-2005/019416
- US-A- 5 600 433
- US-A- 2002 198 483
- US-B1- 6 356 675
- US-B1- 6 631 288

## Description

### FIELD OF THE INVENTION

The present invention relates to a method and means for detecting blood leakage form wounds, and to a corresponding use of the means.

### BACKGROUND OF THE INVENTION

A well-recognised problem in hospital care is that wounds caused by surgery or accidents, in spite of having been properly closed and dressed, may start to bleed again. Due to the dressing by which the wound is covered or due to the patient being unconscious or otherwise unable to recognise the bleeding, it is only noticed by the staff and taken care of after a while. In the meantime the patient may have lost a substantial volume of blood. This will no doubt have a detrimental effect on his or her recovery.

Another problem of similar kind is quite frequently seen in blood dialysis. In a life saving treatment patients with impaired or non-existing renal function purify their blood from salts, urea and other metabolic degradation products on a regular basis, such as two or three times per week. In blood dialysis an artery is punctured by a cannula or needle to make a portion of the patient's blood pass through a dialysis apparatus in which it is purified. The purified blood is returned to the patient by venous infusion through a cannula inserted into a large vein.
Most often arterio-venous fistula (or a corresponding graft) is created at a patient's wrist or upper arm, from which blood is removed by an arterial cannula and returned downstream by a venous cannula.

A cannula of this kind usually comes with wings extending from a short cylindrical plastic tube in which the cannula is mounted. These wings can be used for securing the cannula by the adhesive tape to prevent it from longitudinal displacement in the vein, fistula, or graft. The adhesive tape may accidentally come off and the cannula withdrawn. Inevitably this results in immediate bleeding, which may be quite severe. If the bleeding is not noticed and stopped at once the patient may loose a large volume of blood. Since dialysis patients are usually anaemic, they are particularly affected by such a loss. In addition it is important to prevent blood contained in the dialysis apparatus from being lost if a cannula is removed accidentally. To cope with a loosening arterial needle a safety means is included in known dialysis apparatus. The safety means comprises a pressure sensor disposed on the input side of the apparatus. If the sensor detects a sudden drop in pressure during dialysis the flow of blood through the apparatus is immediately stopped and the personnel alarmed. Due to the pressure drop in the venous needle a loosening thereof cannot be monitored easily in a corresponding manner. The problem of monitoring the insertion state of the venous needle remains to be solved.

The document WO 2005/019416 A2 according to Article 54(3) EPC discloses a method and device for monitoring loss of body fluid and dislodgement of medical instrument from body. In this connection a sensor comprising one or more sensing arrays comprised by one or several optical fibres is disclosed.

US 2002/0198483 A1 relates to an apparatus and a method for detecting the dislodgement of a hyperdemic needle. The known apparatus comprises a resistive and/or capacitive sensor for detecting the wetness of blood and a sensor holder comprising a sterile pad.

WO 01/68163 A2 discloses a system for detecting infusion fluid leaked near an infusion site. The system comprises a chemical capable of reacting with a component in the fluid to form a coloured complex. The coloured complex is formed in a gap disposed between the ends of two optical fiber cables. The site of colour formation is illuminated by a light source connected to one of the cables. The other cable conducts light from the gap to an optical receiver for detecting a change in light caused by formation of the coloured complex. Light passing through the optical fiber cables is not attenuated during its passage.

### OBJECTS OF THE PRESENT INVENTION

One object of the present invention is to provide a method and an apparatus for detecting blood leakage from wounds caused by surgery or accident that have been properly taken care of.

Another object of the present invention is to provide a method and a means for detecting blood leakage from a wound in a large vein upon an accidental removal of a dialysis needle inserted through the wound.

Further objects of the present invention will become obvious from the following summary of the invention, the description of preferred embodiments thereof illustrated in a drawing, and the appended claims.

### SUMMARY OF THE INVENTION

The present invention is based on the insight that blood leakage of the aforementioned kind can be detected by monitoring the passage of light, such as visible or near IR light, through an optical fibre that comprises, at a location intermediate between its first and second ends, a blood detection zone, which has the form of a sharp permanent bend of the fibre. In this application "fibre" always refers to an optical fibre of glass or, more preferred, polymer material that is capable of conducting light efficiently. Preferably a bend of more than 90°, preferably more than 150°, most preferred of about 180° is realized. The sharp bend is a sharp permanent bend that is, one that does substantially retain its form even in absence of coercing force. Optical fibres of polymer material are particularly suitable for being formed into such permanent sharp bends.

In a monitoring state, in which the fibre wall of the blood detection zone is in dry contact with air and/or solid materials, the transport of light in the fibre will not be affected. In an alarm state, in which the fibre wall of the blood detection zone is contacted with blood, the transport of light in the fibre will be notably and even substantially reduced, such as by 10% or 20% or 35% and even up to about 50% or more within a short period of time such as ten seconds and even one second. The reason for this reduction is the change of (inner) reflectance of at a portion of the fibre wall by contact with blood which is a medium of higher refractive index than the fibre material. Specifically the change is from practically total reflection of light conducted by the fibre at the fibre wall in a monitoring state to that of reduced reflection at fibre wall portions that are in contact with blood in an alarm state.

According to an important aspect of the invention the blood detection zone of the fibre is disposed at a flexible patch. The flexible patch is intended for fixation at a wound or a dressing or plaster covering a wound. The wound may be an intentionally caused wound, such as a punctuation of a vein, or one caused accidentally. The means for fixation may be comprised by the patch or be separate. The patch comprises a flexible backing, optionally resilient, of a kind known in the art for surgical patches. Preferably the patch comprises a liquid absorbent capable of soaking blood. Preferably the liquid absorbent material is disposed in contact with the blood detection zone. The absorbent material, which should have good wetting properties, may be of a woven or non-woven kind or a combination thereof. Cotton gauze and cellulose based non-woven materials are suitable absorbents. Preferably the absorbent is disposed on and attached to the underside of the backing, that is, the side that faces the skin in a mounted state of the patch. The patch of the invention may have any suitable form. "Suitable form" relates the body site or dressing site at which the patch is intended to be fixed. Most often the patch will be of about rectangular form. For use with an infusion cannula the patch of the invention preferably comprises an incision extending from its circumference in the direction of its central portion. The inner end of the incision is intended to be disposed adjacent to the cannula.

According to an advantageous aspect of the invention the fibre is firmly fixed at the patch at near its blood detection zone. In this configuration the patch and the fibre form a single disposable device.

According to another advantageous aspect of the invention the patch comprises a short tube of a polymer or other suitable material, preferably a resilient material, fixed to the patch, into which tube the sharp bend of the fibre can be inserted to a depth to make it protrude from the other end of the tube and to be held removably in that position. The openended tube is fixed at the patch by for instance, gluing. More particularly, according to the present invention, is disclosed a means of the aforementioned kind, comprising a light source, a light detector, a patch capable of being fixed to a wound or a wound dressing, an optical fibre being attached to the patch and arranged to conduct light from the light source to the detector in a monitoring state, the light received by the detector being reduced in an alarm state by blood leaking from the wound or the dressing.

According to a first preferred aspect of the present invention is disclosed a means of the aforementioned kind, comprising an apparatus and a single-use disposable device, the apparatus comprising electronic means for the emission and detection of light, in particular visible or near-infrared light, the device comprising a patch, a means for temporary fixation of the patch at a wound or a wound dressing, an optical fibre for conducting the light from the emission means via the patch to the detection means, the fibre at its passage via the patch comprising a zone in which the reflectance of the fibre wall in respect of light conducted by the fibre is affected by blood contacting the wall in the zone, a difference in returned light indicating a leakage of blood or serum from the wound or the dressing.

At its free ends the fibre of the invention is mounted in connectors by which it can be releaseably attached, directly or indirectly, to the light source and the detector, respectively, to put the light source in radiative communication with the detector. The connectors may be of any suitable kind such as, for instance, screw connectors or connectors of a bayonet type or of piston/cylinder type comprising resilient securing means.

According to a further preferred aspect of the invention, the light source and the light detector are disposed in a housing, which additionally comprises coupling means for mounting the light source end and the detector end connectors of the optical fibre mounting means, power supply means, in particular re-chargeable or not re-chargeable cell(s), detector signal amplification means and alarm means. The light of the light source may be continuous. To save power it may also be intermittent, in which case the housing also comprises a light modulating means. The housing further comprises signal amplification means and comparator means for comparing the amplified signal to a signal threshold. If the light received by the detector and amplified falls below the threshold an alarm in the housing is activated electronically. The alarm may emit a sound and or optical signal. The alarm may also emit a radio wave signal and comprise, for this purpose, radio signal emitting means for sending an amplified detector signal or an alarm signal to a remote receiver forming part or being coupled to a central patient monitoring unit or similar in a dialysis clinic, an intensive care department, etc..

### DESCRIPTION OF THE FIGURES

The invention will now be explained in more detail by reference to preferred embodiments illustrated in a drawing in which
Fig. 1 is a side view of a first embodiment of the apparatus of the invention mounted on a forearm of a patient comprising a disposable patch and monitoring unit, in part in section;
Fig. la is a, enlarged partial section of the disposable patch of Fig. 1
Fig. 2 is a sectional view of the patch of the embodiment of Figs. 1 and la;
Fig. 3 is a rough sectional view of the monitoring unit mounted of the apparatus of Figs. 1 to 2;
Fig. 4 is an enlarged partial section of the disposable patch of Figs. 1 and 2, upon the cannula having been accidentally removed;
Fig. 5 is a block scheme of the electronics of the embodiment of Figs. 1 to 4, except for the radiotransmitter/ receiver function.

### DETAILED DESCRIPTION OF A PREFERRED EMBODIMENT

The apparatus of the invention shown in the Figures comprises a single-use disposable flexible patch 1 of about rectangular form. In the Figures it is shown disposed around an infusion cannula 4 inserted through the skin 52 and tissue 53 into a vein 50 in the forearm of a patient. The tip 4' of the cannula 4 is located in the lumen 51 of vein 50. The patch 1 surrounds the cannula 4 at the punctuation site by means of a wedge-formed incision 3 in the patch 1. The incision 3 defines two opposite wings 7, 9 that extend from the central portion of the patch 1. At its distal end the cannula 1 is mounted in a short polycarbonate tube 14 provided with wings (not shown), which stops further insertion of the cannula 4 into the vein 50. From the polycarbonate tube 14 two wings (not shown) extend radially. A flexible tube 6 by which purified blood is adduced from a hemodialysis apparatus (not shown) is in fluid communication with the cannula 4 lumen; neither shown is the corresponding arterial cannula by which blood is transferred from the patient to the hemodialysis apparatus. The cannula 4 and the flexible tube 6 are secured at the patient's arm by adhesive tape, of which only one piece of tape 5 shown. Another piece of adhesive tape (not shown) that secures the cannula 4 is fastened around the cannula wings. The patch 1 includes a flexible backing of a reinforce polymer material, which is provided along its periphery zone 5 with an adhesive layer for releaseably attaching the patch 1 to the skin 52 of the patient. The central portion 13 of the patch 1 is provided with several layers of cotton gauze. A flexible short tube 8 is arranged in the cotton gauze layers so as to extend from the periphery of the patch cotton gauze to a short distance from the inner end of the incision 3. An optical fibre 10, 11, 12 (Toray Industries, Japan; ø 0.25 mm, poly(methylmethacrylate) core, fluorinated polymer cladding) comprising a bend 10 of about 270° and first and second portions 11, 12 extending from either side of the bend 10 is disposed in the tube 8 so as to make the bend 10 extend from the inner end of the tube 8 facing the incision 3, whereas the first and second fibre portions 11, 12 extend from the other, peripheral end of the tube 8 to a monitoring unit 2 comprising a housing 20 disposed at a distance on the patient's forearm on which it is fastened by a bracelet 30. At their free ends the first and second fibre portions 11, 12 are inserted in central bores of cylindrical male parts 15, 16 of snap connections and fixed there by shrinking of the respective male part 15, 16, the corresponding female parts 33, 34 of which are fixed at openings 31, 34 of the housing 20. The female parts 33, 34 are somewhat resilient to allow the somewhat radially bulging front end portions 17, 18 of the male parts 15, 16 to be inserted in the female parts 33, 34 and to be removeably held there. Thus mounted at the monitoring unit 2 the free ends of the first and second fibre portions 11, 12 face a IR diode 21 emitting near IR radiation and a photo diode 22, respectively. The IR diode 21 and the photo diode 22 are electrically connected to an energising circuit 23 and a signal amplification circuit 24, respectively, of an electronics board 25. The circuits 23, 24 are powered by a dry battery 26, which may be rechargeable. The signal amplification circuit comprises a comparator that is designed to detect a sudden fall of the photo detector 22 signal, and is electrically coupled to an alarm unit 27 comprising a bell alarm which is triggered by said fall of signal. Additionally the signal amplification circuit is coupled to a radio wave transmitter 28 comprising an antenna 29. Via the wireless transmitter 28 the monitoring unit 2 is in intermediate or continuous contact with a central monitoring desk 40 of the dialysis department. The monitoring desk 40 comprises a radio wave receiver 41 and an acoustic alarm 42 but may also include, additionally or alternatively, an optical alarm.

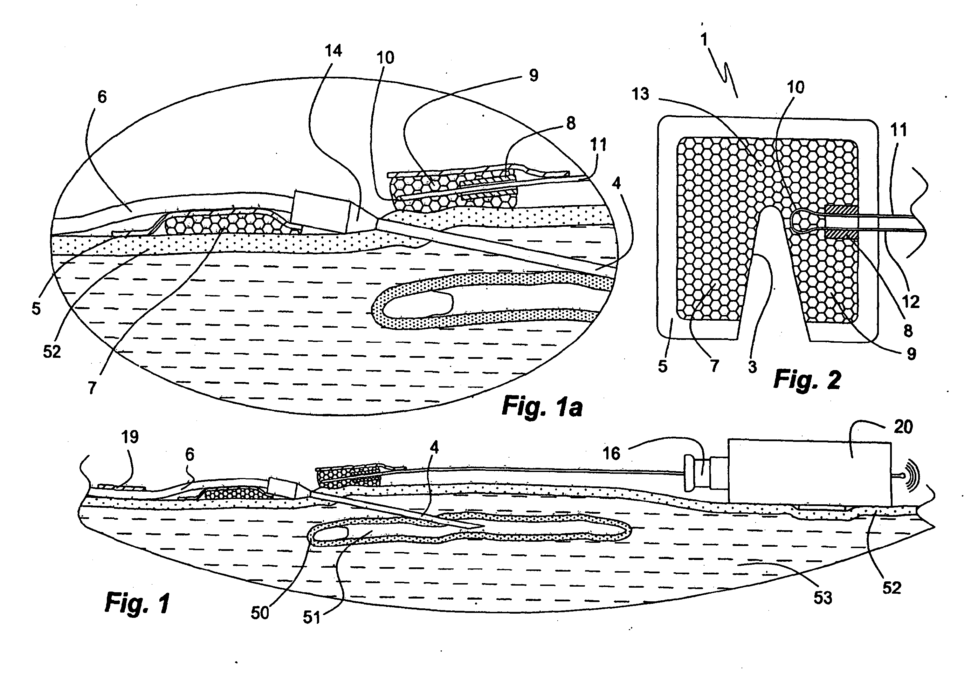

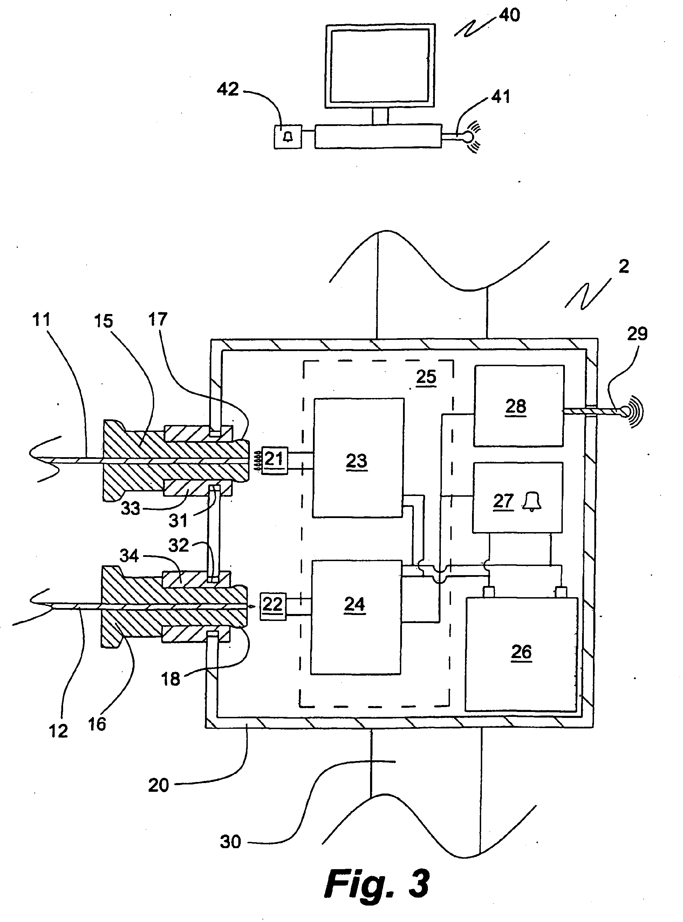

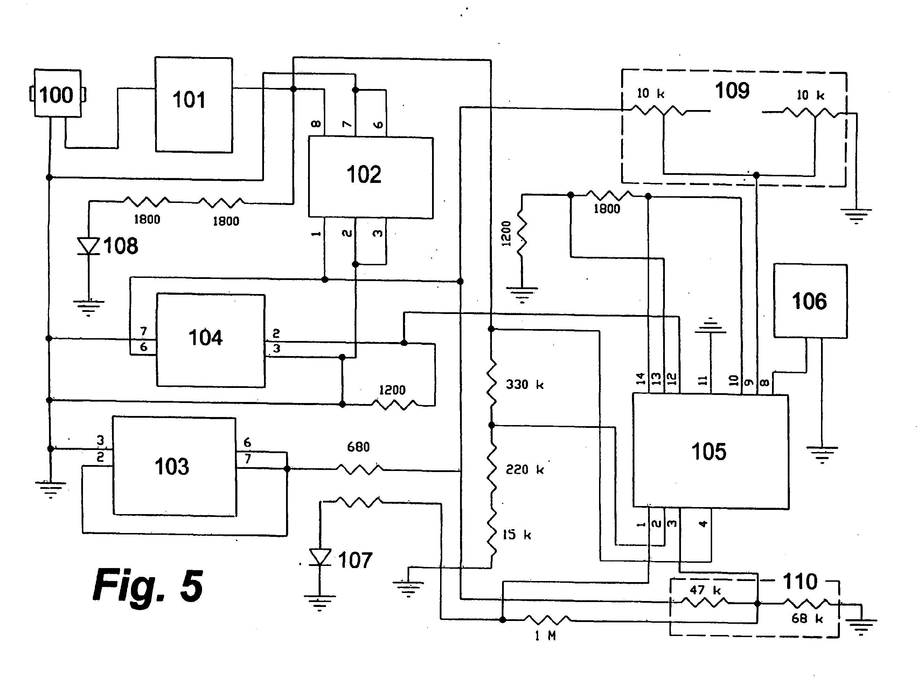

The electronics of the embodiment of Figs. 1 - 3 are illustrated in Fig. 5 except for the wireless transmitter/ receiver function. All resistances are in ohm. Reference nos. designate:
100, dry cell, 9V;
101, on/off switch;
102, DC-DC transformer (3-terminal positive regulator LM78L05ACM 5V, 0.1A; National Semiconductor, U.S.A.), minimum input 6.7 V; 1 = Vₒᵤₜ; 2, 3, 6, 7 = ground; 8 = Vᵢₙ;
103, IR diode (transmitter HFBR-1412, Agilent Technologies, U.S.A.); 2, 6, 7 = anode; 3 = cathode;
104, photodiode light detector (receiver HFBR-2412, Agilent Technologies), incorporates a DC amplifier driving an open-collector Schottky output transistor); 2 = signal out; 3, 7 = ground; 6 = Vᵢₙ;
105, amplifier circuit LM324 (ST Microelectronics, U.S.A.), comprises four independent frequency compensated operational amplifiers (OA); 1 = OA 4, signal out; 2 = OA 4, in (-); 3 = OA 4, in (+); 4 = supply voltage; 5 = OA 3, in (+); 6 = OA 3, in (-); 7 = OA 3, signal out; 8 = OA 2, signal out; 9 = OA 2, in (-); 10 = OA 2, in (+); 11 = ground; 12 = OA 1, in (+); 13 = OA 1 in, (-); 14 = OA 1, signal out. The first OA amplifies the signal by a factor of 1.5; the second OA is comprised by comparator circuit that compares the input signal from OA 1 with a voltage level set by a voltage separator circuit 109 comprising by two pre-set 10 k potentiometers by which either a very low (about 0 V) or a high signal is obtained form OA 2; the signal is further amplified by OA 3 prior to being sent to the signal buzzer. The fourth OA is a comparator for monitoring the circuitry supply voltage from the DC/DC converter that must not decrease below 6.5 V, which is the operation limit for the DC/DC converter 102. The voltage separation by a 47 kΩ/68 kΩ separator circuit 110 is used as a reference. Light emission by diode 107 is triggered by the dry cell voltage falling under 6.7 V;
106, piezoelectric signal buzzer;
107, light-emitting diode, green, 3 mm, EL1224SYGC LED (Everlight, Taiwan); indicates stable operating conditions;
108, light-emitting diode, orange, 3 mm, EL1254USOD/s400 (Everlight, Taiwan); warns for exhausted dry cell.

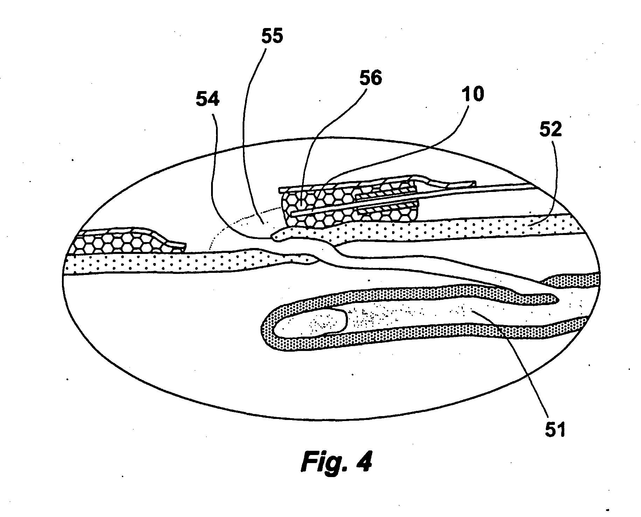

Fig. 4 illustrates the situation immediately after the accidental removal of the venous cannula in Figs. 1 and 1a. Blood 55 emerges from the insertion wound 54. A part 56 of it is soaked up by the cotton layers in the right (seen from below) flap 9 thereby contacting the bend 10 of the optical fibre. The results drop of reflectance in the bend 10 triggers the alarm 27 and sends a radiowave signal to the monitoring desk 40.

## Claims

1. Use of an optical fibre (10, 11, 12) in a state in which visible or infra-red light passes through it, for detecting blood leakage (55) from a wound (54) causing attenuation of the light during its passage, wherein the fibre (10, 11, 12) comprises a blood detection zone comprised by a sharp permanent bend (10) of the fibre (10, 11, 12).

2. The use of claim 1, wherein the light passing trough the fibre (10, 11, 12) is emitted by a light source (21) in radiative communication with one end of the fibre (10, 11, 12) and detected by a photo detector (22) in radiative communication with the other end of the fibre (10, 11, 12).

3. The use of claim 1 or 2, wherein the fibre (10, 11, 12) is of polymer material.

4. The use of any of claims 1 to 3, wherein the photo detector (22) is a photodiode.

5. The use of any of claims 1 to 4, wherein the light source (21) is a light emitting diode.

6. An apparatus for detecting blood leakage (55) from a wound (54), comprising an optical fibre (10, 11, 12), a light source (21) in radiative communication with one end of the fibre (10, 11, 12) and a photo detector (22) in radiative communication with the other end of the fibre (10, 11, 12) and producing an electric signal in response to the light detected, means (24) for amplifying the photo detector signal, means for detecting changes in the amplified signal over time, and alarm means (27), wherein a decrease in signal strength exceeding a pre-set level activates the alarm means (27) to indicate leakage of blood (55), and wherein the optical fibre (10, 11, 12) comprises a blood detection zone disposable near a wound (54) the leakage of which shall be monitored, the blood detection zone being comprised by a sharp permanent bend (10) of the fibre (10, 11, 12).

7. The apparatus of claim 6, wherein the blood detection zone is attached to a patch (1) comprising a blood absorbing material.

8. The apparatus of claim 7, wherein the patch (1) further comprises adhesive means (5) for attaching it to the skin (52) of the patient or to a bandage or similar covering a wound (54).

9. The apparatus of claim 8, wherein the adhesive means (5) is comprised by a flexible backing attached to one side of the patch (1).

10. The apparatus of any of claims 6 to 9, wherein the light source (21), the photo detector (22), the amplifying means (24), the means for detecting changes in the amplified signal and, optionally, the alarm means (27) are disposed in a housing (20) designed to be worn by the patient.

11. The apparatus of claim 10, wherein the housing (20) comprises a power supply means (26) selected from rechargeable or non-rechargeable battery.

12. The apparatus of claim 10 or 11, wherein the optical fibre (10, 11, 12) comprises, at its free ends, means (15, 16) for mounting it to the housing (20) so as to provide for optical communication with the light source (21) and the photo detector (22) through respective openings (31, 32) in the housing (20).

13. The apparatus of any of claims 10 to 12, wherein the housing (20) comprises means (28, 29) for wireless transmission of a signal capable of being received by an alarm (42) disposed at a distance from the patient.

14. A blood leakage detecting device for use in the apparatus of any of claims 6 to 13, comprising an optical fibre (10, 11, 12), wherein the optical fibre comprises a blood detection zone being comprised by a sharp permanent bend (10) of the fibre, means (15, 16) for coupling the fibre (10, 11, 12) to a housing containing a light source (21) and a photo detector (22), the coupling means (21, 22) being disposed at the free ends of the fibre (10, 11, 12), further comprising a patch (1) of a blood absorbing material disposed at sharp permanent bend (10) of the fibre (10, 11, 12).

15. The device of claim 14, further comprising a backing of a thin and flexible material attached to one side of the patch (1), one face of the backing comprising an adhesive means (5) disposed at a circumferential zone thereof surrounding a central zone to which the patch (1) is attached.

16. The device of claim 15, wherein the combination of backing and patch (1) comprises an incision (3) extending from the periphery of the backing towards the centre.

## Patentansprüche

1. Verwendung eines Lichtwellenleiters (10, 11, 12) in einem Zustand, in dem sichtbares Licht oder Infrarotlicht durch ihn hindurchgeht, zum Nachweisen von aus einer Wunde (54) austretendem Blut (55), das eine Abschwächung des Lichts während seines Durchgangs durch den Lichtwellenleiter verursacht, wobei der Lichtwellenleiter (10, 11, 12) eine Zone für den Nachweis von Blut beinhaltet, die durch eine starke dauerhafte Biegung (10) des Lichtwellenleiters (10, 11, 12) umfasst ist.

2. Verwendung nach Anspruch 1, wobei das Licht, das durch den Lichtwellenleiter (10, 11, 12) hindurchgeht, von einer Lichtquelle (21) abgestrahlt wird, die mit einem Ende von dem Lichtwellenleiter (10, 11, 12) in Strahlungsverbindung steht und das von einem Photodetektor (22) nachgewiesen wird, der mit dem anderen Ende von dem Lichtwellenleiter (10, 11, 12) in Strahlungsverbindung steht.

3. Verwendung nach Anspruch 1 oder 2, wobei der Lichtwellenleiter (10, 11, 12) aus Polymermaterial ist.

4. Verwendung nach einem der Ansprüche 1 bis 3, wobei der Photodetektor (22) eine Photodiode ist.

5. Verwendung nach einem der Ansprüche 1 bis 4, wobei die Lichtquelle (21) eine Licht emittierende Diode ist.

6. Gerät zum Nachweisen von austretendem Blut (55) aus einer Wunde (54), umfassend einen Lichtwellenleiter (10, 11, 12), eine Lichtquelle (21), die in Strahlungsverbindung mit einem Ende von dem Lichtwellenleiter (10, 11, 12) steht, und einen Photodetektor (22), der in Strahlungsverbindung mit dem anderen Ende von dem Lichtwellenleiter (10, 11, 12) steht und ein elektrisches Signal als Reaktion auf das erfasste Licht erzeugt, Mittel (24) zur Verstärkung des Photodetektorsignals, Mittel zum Erkennen von Änderungen in dem verstärkten Signal über die Zeit und Alarmmittel (27), wobei eine Abnahme in der Signalstärke, die einen voreingestellten Wert übersteigt, die Alarmmittel (27) aktiviert, um ein Austreten von Blut (55) anzuzeigen, und wobei der Lichtwellenleiter (10, 11, 12) eine Zone für den Nachweis von Blut umfasst, die in der Nähe einer Wunde (54) verfügbar ist, bei der der Blutaustritt überwacht werden soll, wobei die Zone für den Nachweis von Blut durch eine starke dauerhafte Biegung (10) des Lichtwellenleiters (10, 11, 12) umfasst ist.

7. Gerät nach Anspruch 6, wobei die Zone zum Nachweis von Blut mit einer saugfähigen Auflage (1) verbunden ist, die aus einem Blut absorbierenden Material besteht.

8. Gerät nach Anspruch 7, wobei die saugfähige Auflage (1) ferner ein Klebemittel (5) zur Anbringung auf der Haut (52) von dem Patienten oder auf einem Verband oder einer ähnlichen Abdeckung, die eine Wunde (54) bedeckt, umfasst.

9. Gerät nach Anspruch 8, wobei das Klebemittel (5) auf einer flexiblen Unterlageschicht aufgebracht ist, die mit einer Seite von der saugfähigen Auflage (1) verbunden ist.

10. Gerät nach einem der Ansprüche 6 bis 9, wobei die Lichtquelle (21), der Photodetektor (22), die Mittel zum Verstärken (24), die Mittel zum Erkennen von Änderungen in dem verstärkten Signal und, gegebenenfalls, die Alarmmittel (27) in einem Gehäuse (20) angeordnet sind, das für das Tragen durch den Patienten konstruiert ist.

11. Gerät nach Anspruch 10, wobei das Gehäuse (20) ein Stromversorgungsmittel (26) umfasst, das aus wiederaufladbaren oder nichtwiederaufladbaren Batterien ausgewählt ist.

12. Gerät nach Anspruch 10 oder 11, wobei der Lichtwellenleiter (10, 11, 12) an seinen freien Enden Mittel (15, 16) zu seiner Befestigung an dem Gehäuse (20) umfasst, um **dadurch** eine optische Kommunikation mit der Lichtquelle (21) und dem Photodetektor (22) durch die entsprechenden Öffnungen (31, 32) in dem Gehäuse (20) bereitzustellen.

13. Gerät nach einem der Ansprüche 10 bis 12, wobei das Gehäuse (20) Mittel (28, 29) für die drahtlose Übertragung von einem Signal umfasst, das geeignet ist, durch eine Alarmeinheit (42), die in einer Entfernung zu dem Patienten aufgestellt ist, empfangen zu werden.

14. Vorrichtung zum Nachweisen von austretendem Blut zur Verwendung in dem Gerät nach einem der Ansprüche 6 bis 13, umfassend einen Lichtwellenleiter (10, 11, 12), wobei der Lichtwellenleiter eine Zone für den Nachweis von Blut beinhaltet, die durch eine starke dauerhafte Biegung (10) von dem Lichtwellenleiter umfasst ist, Mittel (15, 16) zur Kopplung des Lichtwellenleiters (10, 11, 12) an ein Gehäuse, das eine Lichtquelle (21) und einen Photodetektor (22) enthält, die Kopplungsmittel (21, 22), die an den freien Enden von dem Lichtwellenleiter (10, 11, 12) angeordnet sind, und die ferner eine saugfähige Auflage (1) aus einem Blut absorbierenden Material umfasst, die an der starken dauerhaften Biegung (10) von dem Lichtwellenleiter (10, 11, 12) angeordnet ist.

15. Vorrichtung nach Anspruch 14, die ferner eine Unterlageschicht aus einem dünnen und flexiblen Material umfasst, die mit einer Seite von der saugfähigen Auflage (1) verbunden ist, wobei eine Fläche von der Unterlageschicht ein Klebemittel (5) umfasst, das auf einem umlaufenden Bereich davon angeordnet ist, der einen zentralen Bereich umgibt, welcher mit der saugfähigen Auflage (1) verbunden ist.

16. Vorrichtung nach Anspruch 15, wobei die Kombination aus Unterlageschicht und saugfähiger Auflage (1) einen Einschnitt (3) umfasst, der sich von dem äußeren Rand der Unterlageschicht in Richtung auf das Zentrum erstreckt.

## Revendications

1. Utilisation d'une fibre optique (10, 11, 12) dans un état dans lequel la lumière visible ou infrarouge la traverse, pour détecter un saignement (55) d'une plaie (54) entraînant l'atténuation de la lumière durant son passage, la fibre (10, 11, 12) comprenant une zone de détection du sang composée par un coude permanent (10) de la fibre (10, 11, 12).

2. Utilisation selon la revendication 1, dans laquelle la lumière traversant la fibre (10, 11, 12) est émise par une source de lumière (21) en communication radiative avec une extrémité de la fibre (10, 11, 12) et détectée par un photodétecteur (22) en communication radiative avec l'autre extrémité de la fibre (10, 11, 12).

3. Utilisation selon la revendication 1 ou 2, dans laquelle la fibre (10, 11, 12) est en un matériau polymère.

4. Utilisation selon l'une quelconque des revendications 1 à 3, dans laquelle le photodétecteur (22) est une photodiode.

5. Utilisation selon l'une quelconque des revendications 1 à 4, dans laquelle la source de lumière (21) est une diode électroluminescente.

6. Appareil de détection d'un saignement (55) d'une plaie (54), qui comprend une fibre optique (10, 11, 12), une source de lumière (21) en communication radiative avec une extrémité de la fibre (10, 11, 12) et un photodétecteur (22) en communication radiative avec l'autre extrémité de la fibre (10, 11, 12) et produisant un signal électrique en réponse à la lumière détectée, un moyen (24) destiné à amplifier le signal du photodétecteur, un moyen destiné à détecter des modifications dans le signal amplifié dans le temps, et un moyen d'alarme (27), une diminution de la force du signal au-delà un niveau prédéterminé activant le moyen d'alarme (27) pour indiquer un saignement (55) et la fibre optique (10, 11, 12) comprenant une zone de détection du sang pouvant être disposée à proximité d'une plaie (54) dont le saignement doit être surveillé, la zone de détection de sang étant composée par un coude permanent (10) de la fibre (10, 11, 12).

7. Appareil selon la revendication 6, dans lequel la zone de détection de sang est fixée à un timbre (1) qui comprend un matériau absorbant le sang.

8. Appareil selon la revendication 7, dans lequel le timbre (1) comprend en outre un moyen adhésif (5) pour le fixer à la peau (52) du patient ou à un pansement ou similaire couvrant une plaie (54).

9. Appareil selon la revendication 8, dans lequel le moyen adhésif (5) est composé par un support flexible fixé à un côté du timbre (1).

10. Appareil selon l'une quelconque des revendications 6 à 9, dans lequel la source de lumière (21), le photodétecteur (22), le moyen d'amplification (24), le moyen de détection des modifications du signal amplifié et, facultativement, le moyen d'alarme (27) sont disposés dans un boîtier (20) conçu pour être porté par le patient.

11. Appareil selon la revendication 10, dans lequel le boîtier (20) comprend un moyen d'alimentation en énergie (26) choisi entre une batterie rechargeable et une batterie non rechargeable.

12. Appareil selon la revendication 10 ou 11, dans lequel la fibre optique (10, 11, 12) comprend, au niveau de ses extrémités libres, un moyen (15, 16) destiné à la monter sur le boîtier (20) en vue de fournir une communication optique avec la source de lumière (21) et le photodétecteur (22) par des ouvertures respectives (31, 32) dans le boîtier (20).

13. Appareil selon l'une quelconque des revendications 10 à 12, dans lequel le boîtier (20) comprend un moyen (28, 29) pour la transmission sans fil d'un signal pouvant être reçu par une alarme (42) disposée à une certaine distance du patient.

14. Dispositif de détection de saignement destiné à être utilisé dans l'appareil selon l'une quelconque des revendications 6 à 13, qui comprend une fibre optique (10, 11, 12), la fibre optique comprenant une zone de détection du sang composée par un coude permanent (10) de la fibre, un moyen (15, 16) de couplage de la fibre (10, 11, 12) à un boîtier contenant une source de lumière (21) et un photodétecteur (22), le moyen de couplage (21, 22) étant disposé au niveau des extrémités libres de la fibre (10, 11, 12), et qui comprend en outre un timbre (1) en un matériau absorbant le sang disposé au niveau d'un coude permanent (10) de la fibre (10, 11, 12).

15. Dispositif selon la revendication 14, comprenant en outre un support en un matériau fin et flexible fixé à un côté du timbre (1), une face du support comprenant un moyen adhésif (5) disposé au niveau d'une zone circonférentielle de celui-ci entourant une zone centrale à laquelle le timbre (1) est fixé.

16. Dispositif selon la revendication 15, dans lequel la combinaison du support et du timbre (1) comprend une incision (3) s'étendant de la périphérie du support vers le centre.
